# EUROPEAN PATENT APPLICATION

(11) **EP 4 684 775 A1**
(43) Date of publication of application: **28.01.2026**
(21) Application number: 24386092.1
(22) Date of filing: 25.07.2024
(51) Int. Cl.: A61K 9/00, A61K 9/08, A61K 31/5513, A61K 47/14

(54) **ORAL SOLUTION COMPRISING NITRAZEPAM**

(71) Applicant: LABOMED PHARMACEUTICAL COMPANY S.A., 19441 Koropi, Attica (GR); Roma Pharmaceuticals Ltd., Burton-upon-Trent DE14 2WE (GB)
(72) Inventor: Liolios, Georgios, 15232 Chalandri (GR); Brown, Roland Alexander, Farnham, GU10 1RQ (GB)
(74) Representative: Roukounas, Dimitrios

(57) **Abstract**

An oral pharmaceutical solution comprising nitrazepam and a non-aqueous liquid carrier comprising one or more medium-chain fatty acid triglycerides.

## Description

### FIELD OF THE INVENTION

The present invention relates to oral pharmaceutical solutions comprising nitrazepam.

### BACKGROUND OF THE INVENTION

Nitrazepam (1,3-dihydro-7-nitro-5-phenyl-2H-1,4-benzodiazepin-2-one) is a long-acting benzodiazepine derivative with intermediate onset commonly used to treat panic disorders, severe anxiety, insomnia, and seizures.

Nitrazepam has the following structure.

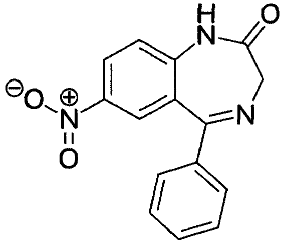

Nitrazepam was first synthesized in the late 1950s by a team of researchers at Hoffmann-La Roche in Switzerland. It was patented in 1961 and came into medical use in 1965.

Robert M. White et. al., in "Detection of Drugs and Their Metabolites in Oral Fluid", 2018 disclose that as a class of compounds, 1,4-benzodiazepines are pharmacodynamically potent but chemically unstable. They demonstrate pH, light, and oxidation sensitivity.

Furthermore, according to GB1218502 it is known that benzodiazepine derivatives, such as nitrazepam, are usually almost insoluble in water but, on the other hand, are readily soluble in various organic solvents. GB1218502 describes in example 2 a non-aqueous solution comprising nitrazepam for parenteral administration, which is produced by dissolving nitrazepam in polyethylene glycol with molecular weight of 300 and filling up with water for injection.

Nitrazepam is currently commercially available as a 5 mg tablet under the brand name Mogadon^{®} among others. It is also commercially available as a "Nitrazepam Mixture 2.5 mg/5 ml Oral Suspension BP", which is an aqueous oral suspension containing as excipients: sucrose, microcrystalline cellulose, carboxymethyl cellulose sodium, mixed esters of p-hydroxybenzoic acid, cherry flavour, and water. According to the Summary of Product Characteristic, the oral suspension should be stored in a refrigerator (2°C - 8°C) and be protected from light.

Drugs/medicines that are required to be kept in the refrigerator are unstable at normal storage conditions of 20 to 25°C and they are called refrigerated drugs/medicines. The fact that the commercially available oral suspension product is a refrigerated drug/medicine, shows that it is challenging to formulate nitrazepam in liquid form.

There is a continuous necessity for drug products with extended shelf life (e.g., longer than twelve months), which is also highlighted by the retail pharmacy practice of routinely returning drug products with remaining shelf lives of less than twelve months. Furthermore, the necessity to have extended shelf lives especially at room temperature (20 to 25°C) is dictated by the fact that refrigeration of drug products increases considerably the cost of treatment, and thus, further reduces the availability of the products to those in need.

The present invention overcomes the problems of the prior art and provides an oral pharmaceutical solution, comprising nitrazepam, which exhibits excellent stability and extended lifetime.

### SUMMARY OF INVENTION

The present invention provides a physicochemically stable oral pharmaceutical solution comprising nitrazepam.

The oral pharmaceutical solution according to the invention comprises nitrazepam and a non-aqueous liquid carrier comprising one or more medium-chain fatty acid triglycerides.

The oral pharmaceutical solution according to the invention exhibits excellent physicochemical stability.

The present invention has the advantage that it provides a stable oral pharmaceutical solution of nitrazepam, by inhibiting hydrolysis and oxidation that typically occur after extended storage.

### DETAILED DESCRIPTION OF THE INVENTION

The present invention provides an oral pharmaceutical solution comprising nitrazepam, in association with a pharmaceutically acceptable non-aqueous liquid carrier.

The term "% w/v" refers to g of the respective substance per 100 ml of the oral solution.

As used throughout the present description and claims, the term "non-aqueous" means essentially water-free.

As used throughout the present description and claims, the term "total impurities" refers to the sum of all nitrazepam degradation impurities present in the oral solution.

The development of an oral solution of nitrazepam is complicated by the fact that the molecule is susceptible to hydrolysis dependent upon the pH value and the water content, and it also demonstrates pH, light, and oxidation sensitivity. Hydrolysis usually leads to the formation of yellow to brownish decomposition products which can strongly color the solutions during storage even under normal storage conditions of 20 to 25°C.

There are numerous lipids comprising triglycerides of fatty acids commercially available to formulators as excipients for lipid-based drug delivery systems. Many synthetic lipids are also available in which the glycerol backbone has been replaced by propylene glycol and/or polyethylene glycols. Additionally, the degree of esterification of the fatty acid moiety may vary, forming mono-, di- and tri-glycerides as well as different esters of propylene glycol and polyethylene glycols. The fatty acids are not necessarily long chain (C₁₁-C₂₂); they can be medium-chain (C₆-C₁₀), short chain (C₁-C₅), unsaturated or branched. Due to these differences in chemical nature, there are numerous lipids or lipid-like excipients available commercially, all of which are colloquially called 'lipids' in the pharmaceutical field.

The term "medium-chain triglycerides" according to the present invention refers to triglycerides of saturated fatty acids having an aliphatic chain of 6 to 10 carbon atoms (C_{6:0} to C_{10:0}).

Unexpectedly, it has been found that the inclusion of many mixed mono-, di- and tri-glycerides, such as glycerol monocaprylocarpate (i.e. medium chain monoglyceride (60% w/w) and diglyceride (35% w/w) consisting of 83% w/w caprylic acid and 17% w/w capric acid) and glycerol dicaprylate (i.e. medium chain diglyceride (83% w/w) comprising 75% - 85% w/w caprylic acid) to oral solutions comprising nitrazepam did not prevent the decomposition of nitrazepam.

It has been further found that many lipophilic surfactants, commonly used in drug carrier systems such as polysorbate 80, polyethylene glycol castor oils and polyethylene glycol hydrogenated castor oils are also ineffective in preventing the decomposition of the active agent after storage.

On the other hand, it has now been found that the physicochemical stability of nitrazepam is considerably enhanced in a non-aqueous liquid carrier comprising one or more medium-chain fatty acid triglycerides. This finding is unexpected since it does not apply to other triglycerides, such as long chain triglycerides. For example, the addition of corn oil, sunflower oil or castor oil does not enhance the stability of nitrazepam in a non-aqueous solution.

Thus, the present invention provides an oral pharmaceutical solution comprising nitrazepam and a non-aqueous liquid carrier comprising one or more medium-chain fatty acid triglycerides.

Preferably, the oral pharmaceutical solution according to the invention comprises from 0.01 % w/v to 0.2 % w/v nitrazepam.

More preferably, the oral pharmaceutical solution according to the invention comprises from 0.02 % w/v to 0.1 % w/v nitrazepam.

The European Pharmacopoeia (Ph. Eur. 6.0) describes medium-chain triglycerides as the fixed oil extracted from the hard, dried fraction of the endosperm of Cocos nucifera L. or from the dried endosperm of Elaeis guineenis Jacq. They comprise a mixture of triglycerides of saturated fatty acids, mainly of caprylic acid (C_{8:0}) and of capric acid (C_{10:0}). They contain no less than 95% w/w of saturated fatty acids (expressed as percent (%) by weight of total mixture).

According to Handbook of excipients, sixth edition (2009), medium-chain triglycerides (synonym: MCT oil) may also be known as fractionated coconut oil, which contains three saturated lipid chains bound to a glycerin backbone and are distinguished from other triglycerides by the length of the carbon chains, normally between 6 and 10.

Medium chain triglycerides have been used in a variety of pharmaceutical formulations including oral, parenteral and topical preparations mainly as emulsifying agents or solvents. They are usually colorless to slightly yellow liquids that are practically odorless and tasteless, while they solidify at about 0°C.

According to a preferred embodiment, the medium-chain triglyceride according to the invention is caprylic triglyceride.

According to another preferred embodiment, the medium-chain triglyceride according to the invention is a mixture of caprylic and capric triglycerides. More preferably, the medium-chain triglyceride according to the invention is a mixture of caprylic and capric triglycerides in a weight ratio caprylic acid : capric acid from 4:6 to 9:1. Even more preferably, the medium-chain triglyceride according to the invention is a mixture of caprylic and capric triglycerides in a weight ratio caprylic acid : capric acid from 4.5:5.5 to 7:1. Most preferably, the medium-chain triglyceride according to the invention is a mixture of caprylic and capric triglycerides in a weight ratio caprylic acid : capric acid from 1:1 to 5:1.

Preferably, the medium-chain triglyceride according to the invention has a saponification value from 320 to 380 mg KOH/g. More preferably, the medium-chain triglyceride according to the invention has a saponification value from 325 to 370 mg KOH/g. Even more preferably, the medium-chain triglyceride according to the invention has a saponification value from 325 to 365 mg KOH/g.

Saponification value or saponification number (SV or SN) represents the number of milligrams of potassium hydroxide (KOH) required to saponify one gram of the medium-chain triglyceride. A high saponification value indicates that the medium-chain triglyceride has a shorter fatty acid chain and a lower molecular weight. A low saponification value indicates that the medium-chain triglyceride has a longer fatty acid chain and a higher molecular weight.

Preferable examples of medium-chain triglycerides according to the invention include
∘ caprylic triglyceride with fatty-acid composition of about 99% w/w caprylic acid (C_{8:0}) (synonyms: tricaprylin, glycerol trioctanoate), with a saponification value of 335 to 360 mg KOH/g, e.g., Captex ^{®} 8000, which is a synthetic triglyceride manufactured by esterification of caprylic acid and glycerin.
∘ caprylic/capric triglyceride with fatty-acid composition of 65% to 80% w/w caprylic acid (C_{8:0}), 20% to 35% w/w capric acid, less than 2% w/w caproic acid (C_{6:0}), less than 2% w/w lauric acid (C_{12:0}) and less than 1% w/w myristic acid (C_{14:0}), with a saponification value of 335 to 355 mg KOH/g e.g. Miglyol ^{®} 810, BergaBest ^{®} MCT Oil 70/30 & Captex ^{®} 300.
∘ caprylic/capric triglyceride with fatty-acid composition of 55% to 70% w/w caprylic acid (C_{8:0}) & 30% to 45% w/w capric acid (C_{10:0}), with a saponification value of 330 to 360 mg KOH/g. e.g. Neobee ^{®} M-5, Cromadol GTCC & Myritol ^{®} 318.
∘ caprylic/capric triglyceride with fatty-acid composition of 50 to 65% caprylic acid (C_{8:0}), 30% to 45% w/w capric acid (C_{10:0}), less than 2% w/w caproic acid (C_{6:0}), less than 2% w/w lauric acid (C_{12:0}) and less than 1% w/w myristic acid (C_{14:0}), with a saponification value of 325 to 345 mg KOH/g e.g. Miglyol ^{®} 812, BergaBest ^{®} MCT Oil 60/40 & Captex ^{®} 355.

The total concentration of the medium-chain triglycerides in the oral pharmaceutical solution according to the invention is at least 55% w/v. Preferably. the total concentration of the medium-chain triglycerides is at least 70% w/v. Even more preferably. the total concentration of the medium-chain triglycerides is at least 95% w/v. Even more preferably. the total concentration of the medium-chain triglycerides is at least 97% w/v.

Preferably, the oral pharmaceutical solution according to the invention is free of sugar alcohols such as, maltitol, glycerol, mannitol, sorbitol, xylitol, erythritol, isomalt and lactitol, as well as polyvinyl alcohol. Additionally, the oral pharmaceutical solution according to the invention is preferably free of ethanol, propylene glycol, polyethylene glycol, polyvinylpyrrolidone, copolyvidone, sorbitan monolaurate, propylene glycol monolaurate (lauroglycol), mixtures of carboxymethyl cellulose with microcrystalline cellulose, as well as free of lipophilic surfactants such as polysorbate 80, polyethylene glycol castor oils and polyethylene glycol hydrogenated castor oils.

Preferably, the oral pharmaceutical solution according to the invention is free of any stabilizing agent which is not a medium chain triglyceride.

The oral pharmaceutical solution, according to the invention may also comprise additional excipients commonly used in preparing oral liquid compositions, such as antimicrobial preservatives, antioxidants, sweeteners and flavouring agents.

Antimicrobial preservatives may include but are not limited to sodium benzoate, benzoic acid, boric acid, sorbic acid and their salts thereof, benzyl alcohol, parahydroxybenzoic acids and their alkyl esters, methyl, ethyl and propyl parahydroxybenzoates and their salts, or mixtures thereof.

Antioxidants which may be used in the present invention comprise, amongst others, butylated hydroxytoluene, butylated hydroxyanisole, ethylenediamine tetraacetic acid ("EDTA"), ascorbic acid, sodium metabisulfite and propyl gallate, or mixtures thereof.

Sweeteners may include but are not limited to aspartame, acesulfame potassium, thaumatin, saccharin and salts thereof, sodium cyclamate, glycyrrhizin, monosodium glycyrrhizinate, monoamonium glycyrrhizinate, or mixtures thereof.

The oral pharmaceutical solution, according to the invention may further comprise flavours and/or colours so as to enhance its palatability and/or visual appearance. Suitable flavouring agents and colouring agents are well known to those skilled in the art. The flavouring agent may be a natural or artificial flavouring agent, including an essence, an extract, a flavour oil, or combinations thereof. Exemplary flavours include, but are not limited to honey flavour, raspberry flavour, strawberry flavour, blueberry flavour, blackberry flavour, grape flavour, peach flavour, apricot flavour, watermelon flavour, melon flavour, fruit punch flavour, cranberry flavour, mango flavour, banana flavour, citrus flavour, orange flavour, lemon flavour, grapefruit flavour, cherry flavour, vanilla flavour, caramel flavour, chocolate flavour, marshmallow flavour, coffee flavour and coconut flavour.

The oral pharmaceutical solution according to the invention is preferably supplied as multidose preparation. Each dose from a multidose container may be administered by means of a device suitable for accurately measuring the prescribed volume. The device is usually a spoon or a cup for volumes of 5 mL or multiples thereof, or an oral syringe for other volumes. Preferably, the device is an oral syringe.

The oral pharmaceutical solution of the present invention may be prepared using methods well known in the art and using regular manufacturing equipment.

For example, it may be prepared using the following process: The active substance and the excipients are weighed. The selected medium-chain triglyceride(s) is added into a vessel and heated to 30 - 35°C. Nitrazepam is added into the vessel under stirring until it totally dissolves. The remaining excipients, if present, are successively added under continuous stirring, until complete dissolution. Finally, the volume is adjusted with a quantity of the medium-chain triglyceride(s).

The final solution is optionally filtered over a 10 µm filter, and filled preferably in light-protective containers, such as amber type III glass 50- or 100-ml bottles sealed with child resistant, tamper evident screw caps.

### EXAMPLES

### EXAMPLE 1

The example shows the improved stability of a solution according to the present invention compared to the stability of a solution according to the prior art (GB1218502). The compositions of the solutions are shown in Table 1 below.

### Trial 1 (according to the present invention)

This nitrazepam composition was prepared in the following manner: Approximately 80% of the total quantity of the selected medium-chain triglyceride was added into a vessel and heated to 30 - 35°C. Nitrazepam was added into the vessel under stirring until it totally dissolved. Finally, the volume was adjusted with the required quantity of the above medium-chain triglyceride.

### Trial 2, (Example 2 of GB1218502)

The nitrazepam composition was prepared in the following manner: 900 g of polyethylene glycol (M.W. 300) was added into a vessel and heated to 30 - 35°C. Nitrazepam was added into the vessel under stirring until it totally dissolved. Finally, the volume was adjusted with the required quantity of water. The final solution was filled in the container (glass bottles).

**Table 1**

| **Component** | **Function** | **Trial 1** | **Trial 2 Ex. 2** GB1218502 |
|---|---|---|---|
| | | **mg/ml** | |
| Nitrazepam | API | 5 | 5 |
| Miglyol 812 ^{®} (50 to 65% (C8:0) / 30% to 45% (C10:0) triglyceride) | Co-solvent | 995 (99.5% w/w) | - |
| PEG 300 | Co-solvent | - | 900 |
| Water | Diluent | - | q.s to 1 ml |

The solutions of Table 1 were stored at 40°C and 75% relative humidity for a period of six months. Quantification of nitrazepam and its degradation impurities was performed by HPLC.

**Table 2**

| | **40°C± 2°C, 75% RH** | |
|---|---|---|
| | **T = 0** | **T = 6 months** |
| **Trial 1** | Assay: 99.1% | Assay: 99.0% |
| | Impurity A: N.D | Impurity A: N.D |
| | Impurity B: N.D | Impurity B: N.D |
| | Any unspecified impurity: N.D. | Any unspecified impurity: N.D. |
| | Total impurities: N.D. | Total impurities: N.D. |
| **Trial 2 Ex. 2** GB1218502 | Assay: 99.8% | Assay: 98.9% |
| | Impurity A: 0.1% | Impurity A: 0.2% |
| | Impurity B: 0.1% | Impurity B: 0.1% |
| | Any unspecified impurity: N.D. | Any unspecified impurity: 0.3% |
| | Total impurities: 0.2% | Total impurities: 0.6% |

| | | |
|---|---|---|
| N.D.: Not detected Nitrazepam impurity A: 3-Amino-6-nitro-4-phenylquinolin-2(1H)-one (as per Eur.Ph.) Nitrazepam impurity B: 5-Amino-2-nitrobenzophenone | | |

The results of Table 2 show that a solution according to the present invention exhibits improved stability of nitrazepam in comparison with the solution of the prior art.

### EXAMPLE 2

The example illustrates the improved stability of nitrazepam in solutions according to the present invention (Compositions I and II) compared to solutions which do not belong to the present invention (Compositions III and IV). The compositions of the solutions are shown in Table 3 below. Glycerol dicaprylate, used in Composition III, is a diglyceride. Glycerol monocaprylocaprate, used in Composition IV, is a monoglyceride.

The nitrazepam solutions were prepared in the following manner:
Approximately 80% of the total quantity of the selected medium-chain triglyceride was added into a vessel and heated to 30 - 35°C. Nitrazepam was added into the vessel under stirring until it totally dissolved. The selected oils or glyceride mixtures were then added under continuous stirring. Finally, the volume was adjusted with the required quantity of the medium-chain triglyceride and/or the oils or glyceride mixtures.

**Table 3**

| | **Composition** | | | |
|---|---|---|---|---|
| | **I** | **II** | **III** | **IV** |
| **Active substance** | **% w/v** | | | |
| Nitrazepam | 0.04 | 0.04 | 0.04 | 0.04 |

| **Excipients** | **% w/v** | | | |
|---|---|---|---|---|
| Miglyol 812 ^{®} (50 to 65% (C_{8:0}) / 30% to 45% (C_{10:0}) triglyceride) | 99.96 | 59.98 | - | - |
| Corn oil | - | 39.98 | - | - |
| Glycerol dicaprylate | - | - | 99.96 | - |
| Glycerol monocaprylocaprate (Capmul MCM ^{®}) | - | - | - | 99.96 |

The solutions of Table 3 were stored at 40°C and 75% relative humidity for a period of six months. Quantification of nitrazepam and its degradation impurities was performed by HPLC.

**Table 4**

| | **40°C± 2°C, 75% RH** | | |
|---|---|---|---|
| | **T** = **0** | **T = 3 months** | **T = 6 months** |
| **Trial I** | Impurity A: N.D. | Impurity A: N.D. | Impurity A: N.D. |
| | Impurity B: N.D. | Impurity B: N.D. | Impurity B: N.D. |
| | Any unspecified impurity: N.D. | Any unspecified impurity: N.D. | Any unspecified impurity: N.D. |
| | Total impurities: N.D. | Total impurities: N.D. | Total impurities: N.D. |
| **Trial II** | Impurity A: N.D. | Impurity A: N.D. | Impurity A: N.D. |
| | Impurity B: N.D. | Impurity B: 0.1% | Impurity B: 0.2% |
| | Any unspecified impurity: N.D. | Any unspecified impurity: 0.1% | Any unspecified impurity: 0.2% |
| | Total impurities: N.D. | Total impurities: 0.2% | Total impurities: 0.4% |
| **Trial III** | Impurity A: N.D. | Impurity A: N.D. | Impurity A: N.D. |
| | Impurity B: N.D. | Impurity B: 0.1% | Impurity B: 0.2% |
| | Any unspecified impurity: N.D. | Any unspecified impurity: 0.2% | Any unspecified impurity: 0.4% |
| | Total impurities: N.D. | Total impurities: 0.3% | Total impurities: 0.6% |
| **Trial IV** | Impurity A: N.D. | Impurity A: N.D. | Impurity A: N.D. |
| | Impurity B: N.D. | Impurity B: 0.1% | Impurity B: 0.2% |
| | Any unspecified impurity: N.D. | Any unspecified impurity: 0.2% | Any unspecified impurity: 0.3% |
| | Total impurities: N.D. | Total impurities: 0.3% | Total impurities: 0.5% |

| | | | |
|---|---|---|---|
| N.D.: Not detected Nitrazepam impurity A: 3-Amino-6-nitro-4-phenylquinolin-2(1H)-one (as per Eur.Ph.) Nitrazepam impurity B: 5-Amino-2-nitrobenzophenone | | | |

The above results show that stability of nitrazepam in a solution according to the present invention is better than the stability exhibited by a solution comprising monoglycerides or diglycerides.

### EXAMPLE 3

Table 5 shows the compositions of oral solutions according to the present invention.

These nitrazepam solutions were prepared in the following manner:
The active substance and the excipients were weighed. Approximately 80% of the total quantity of the selected medium-chain triglyceride(s) was added into a vessel and heated to 30 - 35°C. Nitrazepam was added into the vessel under stirring until it totally dissolved. The flavour was then added under continuous stirring, until complete dissolution. Finally, the volume was adjusted with the required quantity of the medium-chain triglyceride(s).

**Table 5**

| | **Composition** | | |
|---|---|---|---|
| | **A** | **B** | **C** |
| **Active substance** | **% w/v** | | |
| Nitrazepam | 0.04 | 0.04 | 0.05 |

| **Excipients** | **% w/v** | | |
|---|---|---|---|
| Miglyol 812 ^{®} (50 to 65% (Cs:o) / 30% to 45% (C_{10:0}) triglyceride) | 99.76 | 49.88 | - |
| Miglyol 810 ^{®} (65 to 80% (C_{8:0}) / 20% to 35% (C_{10:0}) triglyceride) | - | 49.88 | 99.75 |
| Tutti frutti flavour | 0.2 | 0.2 | 0.2 |

The solutions of Table 5 were stored at 40°C and 75% relative humidity for a period of six months. Quantification of nitrazepam and its degradation impurities was performed by HPLC.

**Table 6: Stability results**

| | **Composition A** | **Composition B** | **Composition C** |
|---|---|---|---|
| **T=0** | | | |
| **Appearance** | Clear, colourless to yellowish solution | Clear, colourless to yellowish solution | Clear, colourless to yellowish solution |
| **Assay** | 101.3% | 99.2% | 99.9% |
| **Impurity A** | N.D. | N.D. | N.D. |
| **Impurity B** | N.D. | N.D. | N.D. |
| **Any unspecified impurity** | N.D. | N.D. | N.D. |
| **Total impurities** | N.D. | N.D. | N.D. |

| **T = 3 months** | | | |
|---|---|---|---|
| **Appearance** | Clear, colourless to yellowish solution | Clear, colourless to yellowish solution | Clear, colourless to yellowish solution |
| **Assay** | 100.3% | 99.2% | 99.9% |
| **Impurity A** | N.D. | N.D. | N.D. |
| **Impurity B** | N.D. | N.D. | N.D. |
| **Any unspecified impurity** | N.D. | N.D. | N.D. |
| **Total impurities** | N.D. | N.D. | N.D. |

| **T = 6 months** | | | |
|---|---|---|---|
| **Appearance** | Clear, colourless to yellowish solution | Clear, colourless to yellowish solution | Clear, colourless to yellowish solution |
| **Assay** | 100.3% | 99.2% | 99.9% |
| **Impurity A** | N.D. | N.D. | N.D. |
| **Impurity B** | N.D. | N.D. | N.D. |
| **Any unspecified impurity** | N.D. | N.D. | N.D. |
| **Total impurities** | N.D. | N.D. | N.D. |

| | | | |
|---|---|---|---|
| N.D.: Not detected Nitrazepam impurity A: 3-Amino-6-nitro-4-phenylquinolin-2(1H)-one (as per Eur.Ph.) Nitrazepam impurity B: 5-Amino-2-nitrobenzophenone | | | |

### EXAMPLE 4

Table 7 shows the compositions of oral solutions according to the present invention. These nitrazepam solutions were prepared as described in previous Example 3.

**Table 7**

| | **Composition** | | | |
|---|---|---|---|---|
| | **D** | **E** | **F** | **G** |
| **Active substance** | **% w/v** | | | |
| Nitrazepam | 0.06 | 0.06 | 0.06 | 0.06 |

| **Excipients** | **% w/v** | | | |
|---|---|---|---|---|
| Captex 8000 ^{®} (∼99% (C_{8:0}) triglyceride) | 99.76 | 49.88 | - | 49.88 |
| Captex 355 ^{®} (50% to 65% (C_{8:0}) / 30% to 45% (C_{10:0}) triglyceride) | - | 49.88 | 99.76 | 49.88 |
| Tutti frutti flavour | 0.2 | 0.2 | 0.2 | 0.2 |

The solutions of Table 7 were stored at 40°C and 75% relative humidity for a period of six months. Quantification of nitrazepam and its degradation impurities, in the compositions prepared, was performed by HPLC.

**Table 8: Stability results**

| | Composition D | **Composition E** | **Composition F** | **Composition G** |
|---|---|---|---|---|
| **T=0** | | | | |
| **Appearance** | Clear, colourless to yellowish solution | Clear, colourless to yellowish solution | Clear, colourless to yellowish solution | Clear, colourless to yellowish solution |
| **Assay** | 101.4% | 99.8% | 100.1% | 100.9% |
| **Impurity A** | N.D. | N.D. | N.D. | N.D. |
| **Impurity B** | N.D. | N.D. | N.D. | N.D. |
| **Any unspecified impurity** | N.D. | N.D. | N.D. | N.D. |
| **Total impurities** | N.D. | N.D. | N.D. | N.D. |

| **T = 3 months** | | | | |
|---|---|---|---|---|
| **Appearance** | Clear, colourless to yellowish solution | Clear, colourless to yellowish solution | Clear, colourless to yellowish solution | Clear, colourless to yellowish solution |
| **Assay** | 100.6% | 99.6% | 99.9% | 100.3% |
| **Impurity A** | N.D. | N.D. | N.D. | N.D. |
| **Impurity B** | N.D. | N.D. | N.D. | N.D. |
| **Any unspecified impurity** | N.D. | N.D. | N.D. | N.D. |
| **Total impurities** | N.D. | N.D. | N.D. | N.D. |

| **T = 6 months** | | | | |
|---|---|---|---|---|
| **Appearance** | Clear, colouriess to yellowish solution | Clear, colourless to yellowish solution | Clear, colourless to yellowish solution | Clear, colourless to yellowish solution |
| **Assay** | 100.8% | 99.3% | 100.3% | 100.0% |
| **Impurity A** | N.D. | N. D. | N.D. | N.D. |
| **Impurity B** | N.D. | N.D. | N.D. | N.D. |
| **Any unspecified impurity** | N.D. | N.D. | N.D. | N.D. |
| **Total impurities** | N.D. | N.D. | N.D. | N.D. |

| | | | | |
|---|---|---|---|---|
| N.D.: Not detected Nitrazepam impurity A: 3-Amino-6-nitro-4-phenylquinolin-2(1H)-one (as per Eur.Ph.) Nitrazepam impurity B: 5-Amino-2-nitrobenzophenone | | | | |

## Claims

1. An oral pharmaceutical solution comprising from 0.01% w/v to 0.2% w/v nitrazepam and a non-aqueous liquid carrier comprising one or more triglycerides of saturated fatty acids having an aliphatic chain of 6 to 10 carbon atoms (medium-chain triglycerides), wherein the total concentration of medium-chain triglycerides in the oral solution is at least 55% w/v.

2. The oral pharmaceutical solution according to claim 1, comprising 0.02% w/v to 0.1% w/v nitrazepam.

3. The oral pharmaceutical solution according to claim 1 or 2, wherein the total concentration of medium-chain triglycerides in the oral solution is at least 70% w/v.

4. The oral pharmaceutical solution according to any one of the preceding claims, wherein the total concentration of medium-chain triglycerides in the oral solution is at least 95% w/v.

5. The oral pharmaceutical solution according to any one of the preceding claims, wherein the total concentration of medium-chain triglycerides in the oral solution is at least 97% w/v.

6. The oral pharmaceutical solution according to any one of the preceding claims, wherein the one or more medium-chain triglycerides is caprylic triglyceride.

7. The oral pharmaceutical solution according to any one of claims 1 to 5, wherein the one or more medium-chain triglycerides is a mixture of caprylic and capric triglycerides.

8. The oral pharmaceutical solution according to any one of claims 1 to 5, wherein the one or more medium-chain triglycerides is a mixture of caprylic and capric triglycerides in a weight ratio caprylic acid : capric acid from 4:6 to 9:1.

9. The oral pharmaceutical solution according to any one of claims 1 to 5, wherein the one or more medium-chain triglycerides is a mixture of caprylic and capric triglycerides in a weight ratio caprylic acid : capric acid from 1:1 to 5:1.

10. The oral pharmaceutical solution according to any one of the preceding claims, wherein the one or more medium-chain triglycerides have a saponification value from 320 to 380 mg KOH/g.

11. The oral pharmaceutical solution according to any one of the preceding claims, wherein the one or more medium-chain triglycerides have a saponification value from 325 to 370 mg KOH/g.

12. The oral pharmaceutical solution according to any one of the preceding claims, wherein the one or more medium-chain triglycerides have a saponification value from 325 to 365 mg KOH/g.

13. The oral pharmaceutical solution according to any one of the preceding claims, wherein the oral solution is free of sugar alcohols, polyvinyl alcohol, ethanol, propylene glycol, polyethylene glycol, polyvinylpyrrolidone, copolyvidone, sorbitan monolaurate, propylene glycol monolaurate (lauroglycol), lipophilic surfactants and mixtures of carboxymethyl cellulose with microcrystalline cellulose.

14. The oral pharmaceutical solution according to claim 13, wherein the sugar alcohol is selected from maltitol, glycerol, mannitol, sorbitol, xylitol, erythritol, isomalt or lactitol.

15. The oral pharmaceutical solution according to any one of the preceding claims, wherein the oral solution is free of any stabilizing agent which is not a medium-chain triglyceride.
